# EUROPEAN PATENT SPECIFICATION

(11) **EP 3 862 753 B1**
(45) Date of publication and mention of the grant of the patent: **07.06.2023**
(21) Application number: 21153742.8
(22) Date of filing: 27.01.2021
(51) Int. Cl.: G01N 33/49, G01N 27/404, A61B 5/157, A61B 5/15

(54) **APPARATUS COMPRISING A FUEL CELL FOR MEASURING A CONCENTRATION OF ALCOHOL**
VORRICHTUNG MIT EINER BRENNSTOFFZELLE ZUR BESTIMMUNG EINER ALKOHOLKONZENTRATION
APPAREIL AVEC UNE PILE À COMBUSTIBLE POUR LA DÉTERMINATION D'UNE CONCENTRATION D'ALCOHOL

(30) Priority: 05.02.2020 GB 202001555
(43) Date of publication of application: 11.08.2021
(73) Proprietor: Surescreen Diagnostics Limited, Derby, Derbyshire DE1 3QB (GB)
(72) Inventor: Campbell, David, Derby, DE1 3QB (GB)
(74) Representative: Swindell & Pearson Limited

(56) References cited:
- US-A1- 2009 182 216
- US-A1- 2011 266 160

## Description

### TECHNOLOGICAL FIELD

Embodiments of the invention relate to detection apparatus, and particularly detection apparatus for determining the concentration of alcohol in a sample.

### BACKGROUND

It is desirable to determine the concentration of alcohol in a sample, for instance, the concentration of alcohol in a blood sample.

It is known to use detection apparatus comprising a swab carrier capable of receiving and presenting a blood sample *via* a capillary which isolates the sample in a test chamber within the detection apparatus. The detection apparatus then conditions the sample so that the equilibrium vapour pressure of the alcohol is generated in the head space according to universal gas laws relating to partial pressure. The concentration of the alcohol vapour at an equilibrium temperature is then measured using a fuel cell by taking into the cell a measured volume from the head space. The blood alcohol content is calculated according to reference to a calibration curve and is read visually or incorporated into a data logging system.

There is a requirement to provide improved detection apparatus capable of more accurately and reproducibly determining the concentration of alcohol in a sample, for instance, the concentration of alcohol in a blood sample.

US 2009/0182216 A1 discloses a method for moisture control in a transdermal blood alcohol monitor.

US 2011/0266160 A1 discloses an alcohol level detection apparatus comprising a chamber associated with a delivery path for a sample volume; conditioning means for the chamber to temperature condition the sample volume in vapour equalisation with air in a head space for the chamber; and a fuel cell to sample the vapour equalisation, the electrical voltage in the fuel cell giving an indication of the alcohol level relative to the temperature condition.

### BRIEF SUMMARY

According an aspect of the invention there is provided detection apparatus for determining the concentration of alcohol in a sample, the detection apparatus comprising:
a collector for receiving a sample, comprising:
   a structure with a recess at one end into which an insert comprising an absorbent material for receiving the sample is locatable; and
   a protective sheath for substantially covering the insert except an area for receiving the sample;
a chamber for receiving at least a part of the collector comprising the sample;
a head space in the chamber when the at least a part of the collector is received in the chamber, wherein the head space is for containing a vapour equalization caused by vapour from the sample equalizing with air in the head space;
a fuel cell associated with the head space;
drawing means to draw the vapour equalization into the fuel cell; and
wherein the collector is configured such that air from outside the detection apparatus is drawn through at least one opening in the collector through at least part of the insert into the head space as the vapour equalization is drawn into the fuel cell.

Possibly, the chamber comprises at least one opening through which air from outside the chamber is drawn into the head space as the vapour equalization is drawn into the fuel cell. The air may be drawn from a body of the detection apparatus.

Possibly, the chamber and the collector are both configured to permit air to be drawn into the head space as the vapour equalization is drawn into the fuel cell. Possibly, the chamber comprises at least one opening through which air from outside the chamber is drawn into the head space as the vapour equalization is drawn into the fuel cell.

The detection apparatus may have a timer to allow for the sample to equilibrate its alcohol level with the air in the head space according to universal gas laws.

The detection apparatus may comprise conditioning means for temperature conditioning the sample in a vapour equalization with air in the head space. The temperature conditioning means may comprise heaters.

The detection apparatus may be configured for determining the concentration of alcohol in a blood sample. The detection apparatus may comprise an electrochemical sensor. The electrochemical sensor may be for blood glucose.

The detection apparatus may comprise a controller having a plurality of algorithms and/or response profiles for the different temperature conditions equating electrical voltage at each temperature condition to alcohol content in the vapour equalization of the sample and air within the head space.

The detection apparatus may be configured so that it purges itself ready for the next analysis operation.

The drawing means may comprise bellows or a volumetric pump.

According to the invention, the collector comprises at least one opening through which air from outside the detection apparatus is drawn into the head space as the vapour equalization is drawn into the fuel cell. The collector comprises a structure with a recess at one end into which an insert comprising an absorbent material for receiving the sample is locatable.

The collector may comprise a removeable protective sheath for substantially covering the insert except an area of absorbent material for receiving the sample.

According to the invention, the collector is configured such that air from outside the detection apparatus is drawn through the at least one opening in the collector through at least part of the insert into the head space as the vapour equalization is drawn into the fuel cell.

According to another aspect of the invention there is provided a method of detecting alcohol in a sample using a detection apparatus, wherein the method comprises:
associating a collector with a chamber, such that the chamber receives at least a part of the collector, wherein the collector comprises:
   a sample;
   a structure with a recess at one end into which an insert comprising an absorbent material for receiving the sample is locatable; and
   a protective sheath for substantially covering the insert except an area for receiving the sample;
containing a vapour equalization caused by vapour from the sample equalizing with air in a head space in the chamber when the at least a part of the collector is received in the chamber;
drawing the vapour equalization into a fuel cell associated with the head space using drawing means; and
drawing air through at least one opening in the collector through at least part of the insert into the head space as the vapour equalization is drawn into the fuel cell.

The detection apparatus, collector and method may comprise any of the features described in any of the preceding statements or following description.

### BRIEF DESCRIPTION

For a better understanding of various examples that are useful for understanding the detailed description, reference will now be made by way of example only to the accompanying drawings in which:
Fig. 1 illustrates a cross sectional view of a detection apparatus comprising a collector;
Fig. 2 illustrates a cross sectional view of the detection apparatus of Fig. 1 but without the collector associated;
Fig. 3 illustrates a cross sectional view of another detection apparatus comprising another collector;
Fig. 4 illustrates a cross sectional view of the detection apparatus of Fig. 3 comprising another collector;
Fig. 5 illustrates a cross sectional view of the detection apparatus of Fig. 1 comprising the collector of Fig. 3;
Fig. 6 illustrates a cross sectional view of the collector of Fig. 3; and
Fig. 7 illustrates a cross sectional view of the collector of Fig. 4.

### DETAILED DESCRIPTION

The figures illustrate a detection apparatus 10 and some example collectors 100 of the detection apparatus 10. The detection apparatus 10 may be referred to as an alcohol detection system.

The detection apparatus 10 according to embodiments of the invention is for determining the concentration of alcohol in a sample. In the illustrated example, the detection apparatus casing 1, which is preferably made from impact resistant plastic such as ABS, houses a display screen. In some examples, the detection apparatus 10 is configured for the value of alcohol in the sample to be displayed digitally, for instance, on the display screen. The detection apparatus casing 1 may also house control switches such as an on/off button and one or more function buttons.

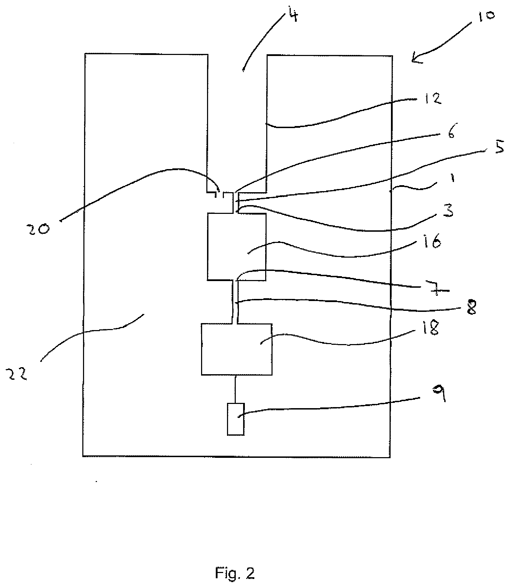

The detection apparatus 10 comprises a collector 100 for receiving a sample, and a chamber 12 for receiving at least a part of the collector 100 comprising the sample. In the illustrated example, the chamber 12 has an opening 4 (see Fig. 2) into which the at least a part of the collector 100 fits snugly.

The detection apparatus 10 comprises a head space 14 in the chamber 12 when the at least a part of the collector 100 is received in the chamber 12. The head space 14 is for containing a vapour equalization caused by vapour from the sample equalizing with air in the head space 14, which could be a partial or full vapour equalization.

The detection apparatus 10 comprises a fuel cell 16 associated with the head space 14, and drawing means 18 to draw the vapour equalization into the fuel cell 16 from the head space 14 to determine the present electrical voltage in the fuel cell 16 indicative of the alcohol level in the vapour equalization relative to a temperature condition. Accordingly, the drawing means 18 is configured to draw a known volume of the vapour equalization into the fuel cell 16, which may be some or all of the volume of the vapour equalization contained in the head space 14.

The term 'drawing means' is intended to cover any means for drawing the vapour equalization into the fuel cell 16 from the head space 14. The vapour equalization may be a gas, a liquid, or a substance comprising gas and liquid.

In the illustrated examples, the drawings means 18 are bellows, as described below. In other examples, the drawings means 18 may comprise any other means for drawing the vapour equalization into the fuel cell 16, for example a volumetric pump.

In other examples, drawing means may be provided to allow a passive dispersion of the vapour equalization across or into the fuel cell by locating the fuel cell inside or adjacent to the chamber 12. In such examples, the drawing means is configured to create an electrical voltage during the gas equalization across or into the fuel cell 16.

In some examples, the detection apparatus 10 comprises at least one sensor to detect gas flow through the detection apparatus 10, i.e. to allow precise determination of the volume of the vapour equalization contained in the head space 14 and/or drawn into the fuel cell 16.

At least the collector 100 is configured to permit air to be drawn into the head space 14 as the vapour equalization is drawn into the fuel cell 16. Advantageously, configuring the collector 100 to permit air to be drawn into the head space 14 as the vapour equalization is drawn into the fuel cell 16 surprisingly allows more accurate and reproducible determination of the concentration of alcohol in a sample. Without being bound by theory, it is believed that permitting air to be drawn into the head space 14 as the vapour equalization is drawn into the fuel cell 16 prevents the occurrence of airlocks, which would otherwise create inconsistencies with the amount of vapour equalization drawn into the fuel cell 16. Accordingly, permitting air to be drawn into the head space 14 as the vapour equalization is drawn into the fuel cell 16 provides better control of the flow of gases through the detection apparatus 10.

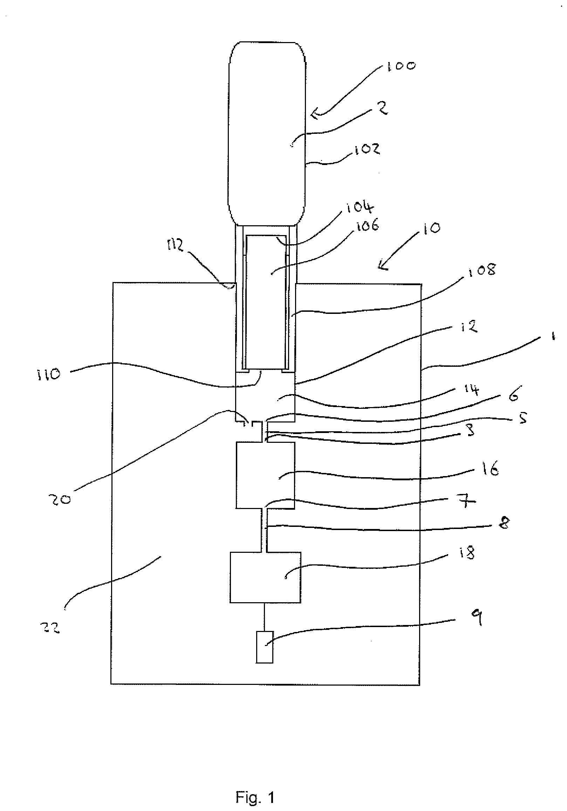

In some examples, for instance as illustrated in Figs. 1, 2, and 5, the chamber 12 also comprises at least one opening 20, i.e. an opening 20 into the chamber 12, through which air from outside the chamber 12 is drawn into the head space 14 as the vapour equalization is drawn into the fuel cell 16. In such examples, the chamber 12 is configured to permit air to be drawn into the head space 14 as the vapour equalization is drawn into the fuel cell 16. The air may be drawn from the body 22 of the detection apparatus 10, i.e. from inside the detection apparatus casing 1. In the illustrated examples, the at least one opening 20 is located in the base of the chamber 12. However, in other examples the at least one opening 20 could be located in the chamber 12 at a different position, for instance, in the side wall of the chamber 12.

In the illustrated examples, the input into the fuel cell 16 is an opening 3, which may be a very small opening. In some examples, a short connecting line 5 may be provided between an output of the chamber 12 and the input into the fuel cell 16. The output from the chamber 12 may be an opening 6, which may also be a very small opening. In other examples, the short connecting line 5 may not be present, such that fuel cell 16 is flush with chamber 12, or a smaller opening is provided directly between the chamber 12 and the fuel cell 16. Accordingly, the chamber 12 and fuel cell 16 are fluidly connected.

In some examples, for instance the illustrated examples, an output from the fuel cell 16 is an opening 7 connected to a tube 8 which is attached to the bellows 18. In some examples, an electrical actuator 9 (for example a solenoid) is connected to the moving piston of the bellows 18.

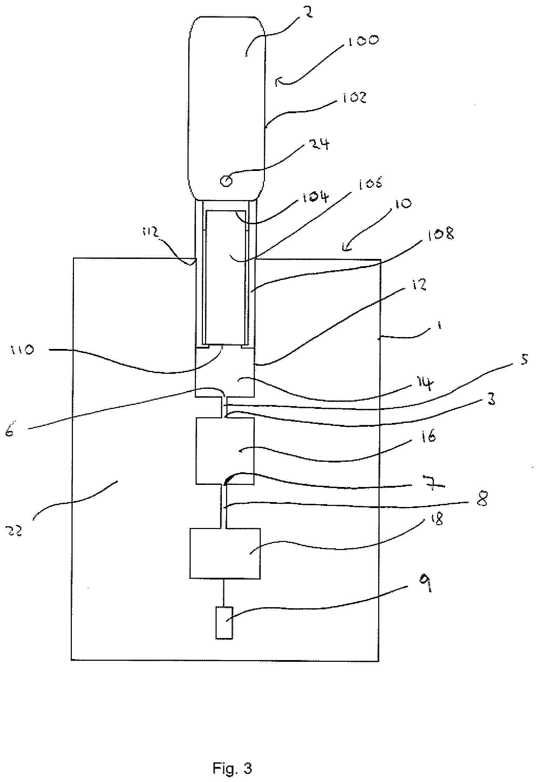

According to the invention, for instance as illustrated in Figs. 3 to 7, the collector 100 comprises at least one opening 24 through which air from outside the detection apparatus 10 is drawn into the head space 14 as the vapour equalization is drawn into the fuel cell 16. According to the invention, the collector 100 is configured to permit air to be drawn into the head space 14 as the vapour equalization is drawn into the fuel cell 16.

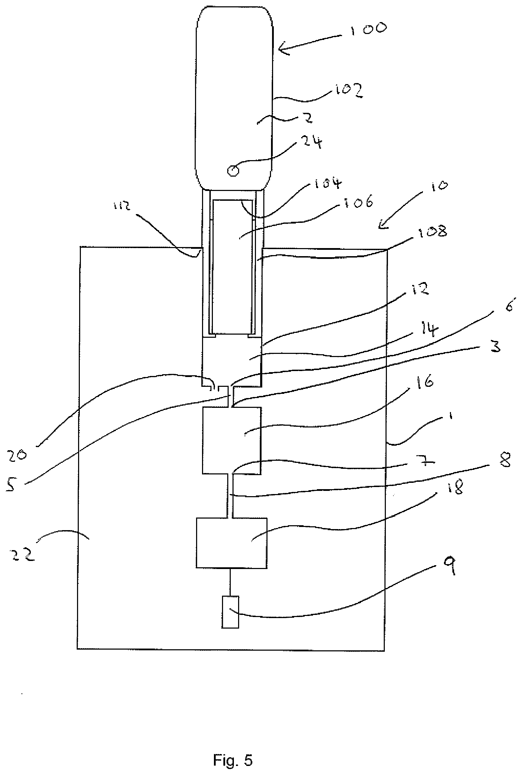

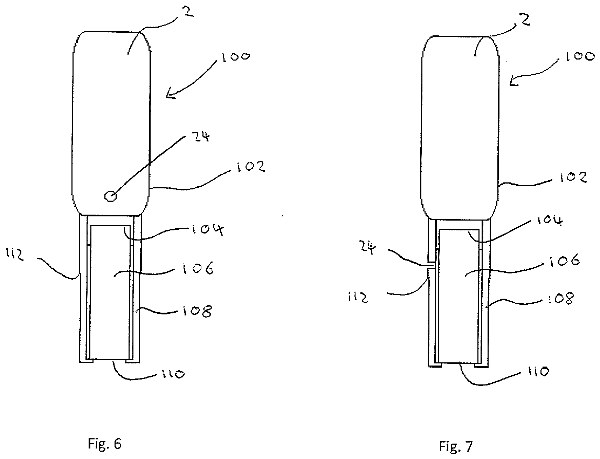

In some examples, for instance as illustrated in Fig. 5, the chamber 12 comprises at least one opening 20 through which air from outside the chamber 12 is drawn into the head space 14 as the vapour equalization is drawn into the fuel cell 16, and the collector comprises at least one opening 24 through which air from outside the detection apparatus 10 is drawn into the head space 14 as the vapour equalization is drawn into the fuel cell 16. In such examples, the chamber 12 and the collector 100 are both configured to permit air to be drawn into the head space 14 as the vapour equalization is drawn into the fuel cell 16. The collector 100 illustrated in Fig. 5 is of the type illustrated in Fig. 6. Alternatively, the collector 100 could be of the type illustrated in Fig. 7, or any other collector 100 according to embodiments of the invention.

The detection apparatus 10 may have a timer to allow for the sample to equilibrate its alcohol level with the air in the head space 14 according to universal gas laws. In some examples, the head space 14 has a volume no greater than 1.0 ml for a 30 microlitre sample.

In some examples, the detection apparatus 10 comprises conditioning means for temperature conditioning the sample in a vapour equalization with air in the head space 14. The temperature conditioning means may comprise heaters, for instance, located in the body 22 of the detection apparatus 10 proximal to the chamber 12 and/or fuel cell 16. In some examples, the detection apparatus 10 also comprises a thermistor or thermocouple to control the temperature in the chamber 12 and/or fuel cell 16.

When present, the purpose of the heaters is to provide stable conditions for the analysis under all practical situations, because it is envisaged that the detection apparatus 10 could even be employed in sub-zero conditions such as at road accidents in Scandinavia and the like. The partitioning of alcohol into the head space 14 may be affected by temperature and so it is desirable to be aware of the temperature at the time of the test.

In some examples of the test it is foreseen that the detection apparatus 10 could be used substantially at ambient room temperature (such as in a hospital emergency room). In this case the detection apparatus 10 may be equipped with sets of calibration data at a range of temperatures. The detection apparatus 10 thermistor or thermocouple (if present) or similar sensing device allows the detection apparatus 10 to choose the appropriate calibration curve for that temperature and provide an accurate alcohol result at a range of temperatures. In this case heaters may be dispensed with. Accordingly, the detection apparatus 10 may comprise a controller having a plurality of algorithms and/or response profiles or formulas for the different temperature conditions equating electrical voltage at each temperature condition to alcohol content in the vapour equalization of the sample and air within the head space 14. In some examples, computer processing against a formula may be required.

In some examples, the detection apparatus 10 is configured for determining the concentration of alcohol in a blood sample. In such examples, the detection apparatus 10 may comprise an electrochemical sensor for blood glucose. Accordingly, the detection apparatus 10 may display blood glucose and blood alcohol simultaneously. There are distinct advantages in some emergency medicinal situations (such as coma or collapse) to have readings of both blood glucose and blood alcohol simultaneously. Other parameters and biomarkers may be also be screened within the device, displaying a result upon analysis.

In some examples, the detection apparatus 10 may be incorporated into automatic detections systems such as at blood transfusion centres, and routine blood monitoring systems within a hospital or a drug and alcohol rehabilitation clinic, or in locations such as intensive care units, critical care or cardiovascular units where consumption of alcohol is not permitted.

In some examples, the sample may be an alcoholic beverage. In such examples, the level of alcohol determined to be present in a sample may be used to determine the authenticity of the alcoholic beverage and/or the alcohol proof of the beverage.

In some examples, the body 22 of the detection apparatus 10 has compartments for batteries, which may be disposable or rechargeable batteries. Where a rechargeable detection apparatus 10 is preferred, contacts are provided to allow the detection apparatus 10 to be placed in a cradle for convenient storage when the detection apparatus 10 will automatically be recharged.

The detection apparatus 10 may be set to accommodate the particular conditions required by the operator, using the one or more function buttons to toggle through one or more programs stored in a memory. For example, pushing the on/off button turns the detection apparatus 10 on, then if a function button is pushed within three seconds this puts the detection apparatus 10 into set up mode. The one or more function buttons may be used to toggle through different programs, for example, for testing time, display units (for example milligrams/100 ml), whether the detection apparatus 10 should be back-lit, and whether the detection apparatus 10 should provide a torch facility in situations (such as a road accident) where visibility may be poor. In accident situations, a short conditioning time may be chosen since speed is more important than absolute accuracy, while in a hospital environment or where a patient's alcohol level is being monitored periodically, absolute accuracy may be more important than speed of analysis, in which case a longer conditioning time may be chosen.

The conditioning time, period or stage is the time allowed for vapour from the sample to equalize with air in the head space 14 to provide the vapour equalization. This conditioning time may be changed depending on variables detected by the device or selected by the user.

Once the chosen option is displayed on the display, a start button may select that option. After the chosen option, or if a function button is not pressed, the detection apparatus 10 may be configured to go through a conditioning stage of purging (and in some examples heating) in preparation for receiving the collector 100 comprising the sample in the chamber 12.

Accordingly, in some examples the detection apparatus 10 is configured so that it purges itself ready for the next analysis operation.

If no action is taken during the toggle, the detection apparatus 10 may be configured to turn off automatically after a period of inactivity, such as after 30 seconds.

According to the invention, the collector 100 comprises a structure 102 with a recess 104 at one end into which an insert 106 comprising an absorbent material for receiving the sample is locatable. The insert 106 may be removable and disposable. It is envisaged that a new insert 106 may be used for each new sample.

The structure 102 may be moulded from a plastics material, such as polystyrene. The structure 102 may be constructed from hollow tube to minimise the amount of plastics used, and it may conveniently be produced with a handle 2, for example, an integrally formed handle 2 such as in the illustrated examples. The handle 2 may comprise ridges making it easier to handle wearing latex gloves.

In the illustrated examples, the collector 100 comprises a removeable protective sheath 108 for substantially covering the insert 106 except an area 110 for receiving the sample. In other examples, the protective sheath 108 is not removeable. The protective sheath 108 may be transparent enabling facile and accurate collection of a sample.

The absorbent material may comprise cellulose acetate. The absorbent material may be inert. The absorbent material may be a spongy fabric or membrane. The absorbent material of the insert 106 provides a large surface area on which to locate the sample, which increases the rate at which vapour from the sample equalizes with air in the head space 14. This increases the speed of calibration and hence accuracy of detection at a given conditioning time.

According to the invention, the collector 100 is configured such that air from outside the detection apparatus 10 is drawn through the at least one opening 24 in the collector 100 and then through at least part of the insert 106 into the head space 14 as the vapour equalization is drawn into the fuel cell 16. The insert 106 is permeable to the flow of air. The flow of air through the at least part of the insert 106 into the head space 14 (and therefore through the sample) increases the amount of alcohol extracted from the sample, and thus improves the accuracy of detection of alcohol in the sample.

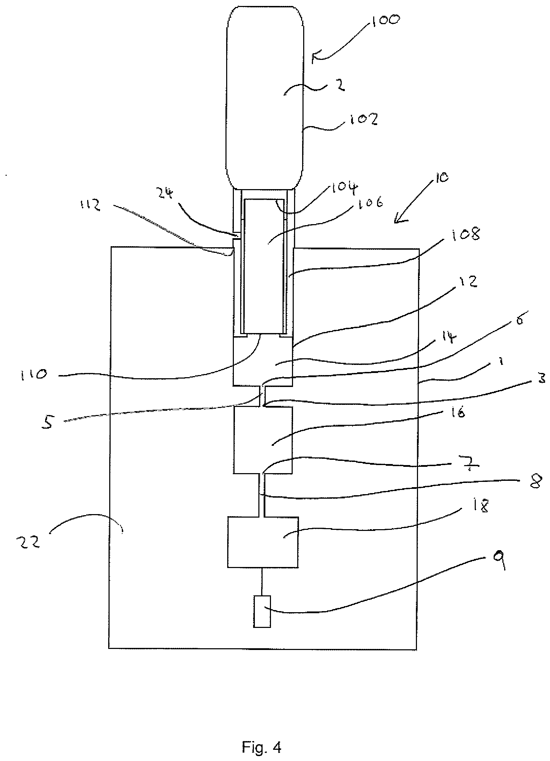

The at least one opening 24 may be provided extending through the structure 102 to the insert 106 at or proximal to the handle 2 (as illustrated in Figs. 3, 5, and 6). Alternatively, the at least one opening 24 may be provided extending through the sheath 108 to the insert 106 (as illustrated in Figs. 4 and 7).

The collector 100, and in particular the insert 106, and most particularly the area 110 for receiving a sample, may comprise and/or is coated with a blood anti-clotting agent such as EDTA or sodium fluoride to prevent a blood sample from clotting and skinning over.

In some examples, in a condition wherein the collector 100 is received in the chamber 12, the area 110 of absorbent material is about 3 mm away from the output 6 of the chamber 12. The chamber 12 and/or collector 100 may include a formation to limit the extent the collector 100 extends in to the chamber 12 in use, and thus to define the distance the area 110 of the absorbent material is away from the output 6 of the chamber 12. In the illustrated examples, the collector 100 comprises a circumferential lip 112 to limit the extent to which the collector 100 extends into the chamber 12. The protective sheath 108 may comprise the circumferential lip 112.

Embodiments of the invention also provide a method of detecting alcohol in a sample using a detection apparatus 10.

The method comprises associating a collector 100 comprising a sample with a chamber 12, such that the chamber 12 receives at least a part of the collector 100.

The method further comprises containing a vapour equalization caused by vapour from the sample equalizing with air in a head space 14 in the chamber 12 when the at least a part of the collector 100 is received in the chamber 12.

The method further comprises drawing the vapour equalization (which may be full or partial vapour equalization) into a fuel cell 16 associated with the head space 14 using drawing means 18 to determine the present electrical voltage in the fuel cell 16 indicative of the alcohol level in the vapour equalization relative to a temperature condition. As described above, the drawing means 18 in the illustrated example are bellows 18.

The method further comprises drawing air into the head space 14 as the vapour equalization is drawn into the fuel cell 16, which may be a predetermine volume of air.

An example of the detection apparatus 10 of the illustrated examples, in use, is now described in relation to a blood sample. To collect a sample, a donor's finger is chosen and a standard 30 microlitre lancet is used to draw blood. This activity is common in medical applications and is similar to that used to collect blood for a glucose determination. Once a hanging droplet of blood is available the collector 100 is applied to the finger and the blood is absorbed onto the area 110 of the insert 106.

The detection apparatus 10 is then turned on and undergoes a countdown period to prepare the detection apparatus 10. The collector 100 comprising the sample is then pushed into the chamber 12 and the detection apparatus 10 starts its conditioning period. In some examples, the conditioning period may require activation of the heater controlled by its thermistor or thermocouple. In other examples, heating is not required. A timer starts which continues for the pre-set conditioning time. Experimentation has shown that 45 seconds is sufficient for an approximate analysis (such as at an accident scene) while up to 3 minutes provides a more accurate analysis. During conditioning the alcohol in the blood sample outgases and develops an equilibrium with the air in the head space 14, according to universal gas laws, which may be at the temperature given by the heater controlled by its thermistor or thermocouple or according to a measured temperature without heating. This alcohol is prevented from entering the fuel cell 16 by virtue of the small opening 3 and the volume of air already within the fuel cell 16, thereby minimising diffusion of conditioned air into the fuel cell 16.

Once the conditioning time has elapsed, in the illustrated examples the timer activates the actuator 9 drawing air from the tube 8 into the bellows 18, which simultaneously displaces conditioned air (i.e. the vapour equalization) from the head space 14 into the fuel cell 16 so that the fuel cell 16 senses the level of alcohol in the conditioned air (i.e. in the vapour equalization). This generates an electrical voltage which is proportional to the quantity of alcohol in the conditioned air (i.e. in the vapour equalization). Electronics within the detection apparatus 10 interpolates the alcohol value from a calibration curve and displays it on the display, for example, as a % by volume or mmol/l or any other appropriate measure selected.

At the same time, air is drawn into the head space 14 through opening 20 and/or opening 24. In examples in which air is draw through opening 24 (partially or exclusively), the air drawn into the head space 14 through the material of insert 106.

In some examples, the detection apparatus 10 may be used for detection of alcohol in a natural head space such as exists above grain silos, fermentation vessels, food stores, and the like. Here the detection apparatus 10 is fitted with a fine aspiration needle preferably with a pointed tip, so that it is able to penetrate a septum provided into the vessel or container for the purpose of inserting the needle. In the illustrated examples, the detection apparatus 10 then collects a sample from the head space by operating the actuator 9 several times so that conditioned air from the head space is drawn into the fuel cell 16 aided by a one-way valve in the tube 8 ensuring only one-way flow as the bellows 18 cycles. Detection of alcohol in such circumstances may be valuable in early detection of process difficulties such as yeast attack of foodstuffs, or monitoring of a fermentation process.

Embodiments of the invention provide a number of advantages as detailed above.

Although embodiments of the present invention have been described in the preceding paragraphs with reference to various examples, it should be appreciated that modifications to the examples given can be made without departing from the scope of the invention as claimed. For example, the detection apparatus 10 may comprise a semi- permeable membrane, such as Gore-Tex^{®} located, for example, in the chamber 12, and/or proximal to the inlet 3 to the fuel cell 16 to prevent non-gaseous contaminants from entering the fuel cell 16.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term "example" or "for example" or "may" in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a sub-class of the class that comprise some but not all of the instances in the class. It is therefore implicitly disclosed that a feature described with reference to one example but not with reference to another example, can where possible be used in that other example but does not necessarily have to be used in that other example.

## Claims

1. Detection apparatus (10) for determining the concentration of alcohol in a sample, the detection apparatus comprising:
a collector (100), for receiving a sample, comprising:
a structure (102) with a recess (104) at one end into which an insert (106) comprising an absorbent material for receiving the sample is locatable; and
a protective sheath (108) for substantially covering the insert except an area for receiving the sample;
a chamber (12) for receiving at least a part of the collector comprising the sample;
a head space (14) in the chamber when the at least a part of the collector is received in the chamber, wherein the head space is for containing a vapour equalization caused by vapour from the sample equalizing with air in the head space;
a fuel cell (16) associated with the head space;
drawing means (18) to draw the vapour equalization into the fuel cell; and
wherein the collector is configured such that air from outside the detection apparatus is drawn through at least one opening (24) in the collector through at least part of the insert into the head space as the vapour equalization is drawn into the fuel cell.

2. A detection apparatus according to claim 1, wherein the chamber comprises at least one opening (20) through which air from outside the chamber is drawn into the head space as the vapour equalization is drawn into the fuel cell.

3. A detection apparatus according to claim 2, wherein the air is drawn from a body (22) of the detection apparatus.

4. A detection apparatus according to any of the preceding claims, wherein the chamber and the collector are both configured to permit air to be drawn into the head space as the vapour equalization is drawn into the fuel cell, wherein the chamber comprises at least one opening (20) through which air from outside the chamber is drawn into the head space as the vapour equalization is drawn into the fuel cell, and the collector comprises at least one opening through which air from outside the detection apparatus is drawn into the head space as the vapour equalization is drawn into the fuel cell.

5. A detection apparatus according to any of the preceding claims, wherein the detection apparatus has a timer to allow for the sample to equilibrate its alcohol level with the air in the head space according to universal gas laws.

6. A detection apparatus according to any of the preceding claims, wherein the detection apparatus comprises conditioning means for temperature conditioning the sample in a vapour equalization with air in the head space, wherein the temperature conditioning means comprises heaters.

7. A detection apparatus according to any of the preceding claims, wherein the detection apparatus comprises an electrochemical sensor, wherein the electrochemical sensor is for blood glucose.

8. A detection apparatus according to any of the preceding claims, wherein the detection apparatus comprises a controller having a plurality of algorithms and/or response profiles for the different temperature conditions equating electrical voltage at each temperature condition to alcohol content in the vapour equalization of the sample and air within the head space.

9. A detection apparatus according to any of the preceding claims, wherein the detection apparatus is configured so that it purges itself ready for the next analysis operation.

10. A detection apparatus according to any of the preceding claims, wherein the drawing means are bellows or a volumetric pump.

11. A method of detecting alcohol in a sample using detection apparatus (10), wherein the method comprises:
associating a collector (100) with a chamber (12), such that the chamber receives at least a part of the collector, wherein the collector comprises:
a sample;
a structure (102) with a recess (104) at one end into which an insert (106) comprising an absorbent material for receiving the sample is locatable; and
a protective sheath (108) for substantially covering the insert except an area for receiving the sample;
containing a vapour equalization caused by vapour from the sample equalizing with air in a head space (14) in the chamber when the at least a part of the collector is received in the chamber;
drawing the vapour equalization into a fuel cell (16) associated with the head space using drawing means (18); and
drawing air through at least one opening (24) in the collector through at least part of the insert into the head space as the vapour equalization is drawn into the fuel cell.

## Patentansprüche

1. Detektionsvorrichtung (10) zur Bestimmung der Alkoholkonzentration in einer Probe, wobei die Detektionsvorrichtung Folgendes aufweist:
einen Sammler (100) zur Aufnahme einer Probe, aufweisend:
eine Struktur (102) mit einer Ausnehmung (104) an einem Ende, in der ein ein absorbierendes Material zur Aufnahme der Probe aufweisender Einsatz (106) anordenbar ist; und
eine Schutzhülle (108), die den Einsatz im Wesentlichen abdeckt, mit Ausnahme eines Bereichs zur Aufnahme der Probe;
eine Kammer (12) zur Aufnahme mindestens eines Teils des die Probe aufweisenden Sammlers;
einen Kopfraum (14) in der Kammer, wenn der mindestens eine Teil des Sammlers in der Kammer aufgenommen ist, wobei der Kopfraum dazu dient, einen Dampfausgleich zu enthalten, der dadurch verursacht wird, dass Dampf aus der Probe mit Luft in dem Kopfraum ausgeglichen wird;
eine mit dem Kopfraum verbundene Brennstoffzelle (16);
Ansaugmittel (18) zum Ansaugen des Dampfausgleichs in die Brennstoffzelle; und
wobei der Sammler so konfiguriert ist, dass Luft von außerhalb der Detektionsvorrichtung durch mindestens eine Öffnung (24) im Sammler durch mindestens einen Teil des Einsatzes in den Kopfraum gesaugt wird, während der Dampfausgleich in die Brennstoffzelle gesaugt wird.

2. Detektionsvorrichtung nach Anspruch 1, **dadurch gekennzeichnet, dass** die Kammer mindestens eine Öffnung (20) aufweist, durch die Luft von außerhalb der Kammer in den Kopfraum gesaugt wird, während der Dampfausgleich in die Brennstoffzelle gesaugt wird.

3. Detektionsvorrichtung nach Anspruch 2, **dadurch gekennzeichnet, dass** die Luft aus einem Körper (22) der Detektionsvorrichtung angesaugt wird.

4. Detektionsvorrichtung nach einem der vorhergehenden Ansprüche, **dadurch gekennzeichnet, dass** die Kammer und der Sammler beide so konfiguriert sind, dass Luft in den Kopfraum gesaugt werden kann, wenn der Dampfausgleich in die Brennstoffzelle gesaugt wird, wobei die Kammer mindestens eine Öffnung (20) aufweist, durch die Luft von außerhalb der Kammer in den Kopfraum gesaugt wird, wenn der Dampfausgleich in die Brennstoffzelle gesaugt wird, und der Sammler mindestens eine Öffnung aufweist, durch die Luft von außerhalb der Detektionsvorrichtung in den Kopfraum gesaugt wird, wenn der Dampfausgleich in die Brennstoffzelle gesaugt wird.

5. Detektionsvorrichtung nach einem der vorhergehenden Ansprüche, **dadurch gekennzeichnet, dass** die Detektionsvorrichtung einen Zeitgeber hat, der es der Probe ermöglicht, ihren Alkoholgehalt mit der Luft im Kopfraum gemäß den universellen Gasgesetzen ins Gleichgewicht zu bringen.

6. Detektionsvorrichtung nach einem der vorhergehenden Ansprüche, **dadurch gekennzeichnet, dass** die Detektionsvorrichtung Konditionierungsmittel zur Temperaturkonditionierung der Probe bei einem Dampfausgleich mit der Luft im Kopfraum aufweist, wobei die Temperaturkonditionierungsmittel Heizelemente aufweisen.

7. Detektionsvorrichtung nach einem der vorhergehenden Ansprüche, **dadurch gekennzeichnet, dass** die Detektionsvorrichtung einen elektrochemischen Sensor aufweist, wobei der elektrochemische Sensor für Blutglukose ist.

8. Detektionsvorrichtung nach einem der vorhergehenden Ansprüche, **dadurch gekennzeichnet, dass** die Detektionsvorrichtung eine Steuerungseinrichtung mit einer Vielzahl von Algorithmen und/oder Ansprechprofilen für die verschiedenen Temperaturbedingungen aufweist, die die elektrische Spannung bei jeder Temperaturbedingung mit dem Alkoholgehalt im Dampfausgleich der Probe und der Luft im Kopfraum gleichsetzt.

9. Detektionsvorrichtung nach einem der vorhergehenden Ansprüche, **dadurch gekennzeichnet, dass** die Detektionsvorrichtung so konfiguriert ist, dass sie sich selbst spült, um für den nächsten Analysevorgang bereit zu sein.

10. Detektionsvorrichtung nach einem der vorangehenden Ansprüche, **dadurch gekennzeichnet, dass** die Ansaugmittel Faltenbälge oder eine volumetrische Pumpe sind.

11. Verfahren zum Nachweis von Alkohol in einer Probe unter Verwendung einer Detektionsvorrichtung (10), **dadurch gekennzeichnet, dass** das Verfahren aufweist:
Zuordnen eines Sammlers (100) zu einer Kammer (12), so dass die Kammer zumindest einen Teil des Sammlers aufnimmt, wobei der Sammler aufweist:
eine Probe;
eine Struktur (102) mit einer Ausnehmung (104) an einem Ende, in der ein ein absorbierendes Material zur Aufnahme der Probe aufweisender Einsatz (106) anordenbar ist; und
eine Schutzhülle (108), die den Einsatz im Wesentlichen abdeckt, mit Ausnahme eines Bereichs zur Aufnahme der Probe;
Aufnehmen eines Dampfausgleichs, der dadurch verursacht wird, dass sich Dampf aus der Probe mit Luft in einem Kopfraum (14) in der Kammer ausgleicht, wenn der mindestens eine Teil des Sammlers in der Kammer aufgenommen ist;
Ansaugen des Dampfausgleichs in eine Brennstoffzelle (16), die mit dem Kopfraum verbunden ist, unter Verwendung von Ansaugmitteln (18); und
Ansaugen von Luft durch mindestens eine Öffnung (24) in den Sammler durch mindestens einen Teil des Einsatzes in den Kopfraum, wenn der Dampfausgleich in die Brennstoffzelle gesaugt wird.

## Revendications

1. Appareil de détection (10) pour déterminer la concentration d'alcool dans un échantillon, l'appareil de détection comprenant :
un collecteur (100), pour recevoir un échantillon, comprenant :
une structure (102) ayant un évidement (104) à une extrémité dans lequel un insert (106) comprenant un matériau absorbant pour recevoir l'échantillon peut être placé ; et
une gaine de protection (108) pour recouvrir sensiblement l'insert à l'exception d'une zone destinée à recevoir l'échantillon ;
une chambre (12) pour recevoir au moins une partie du collecteur comprenant l'échantillon ;
un espace de tête (14) dans la chambre lorsque ladite au moins une partie du collecteur est reçue dans la chambre, l'espace de tête étant destiné à contenir une égalisation de vapeur provoquée par la vapeur provenant de l'échantillon s'équilibrant avec l'air dans l'espace de tête ;
une pile à combustible (16) associée à l'espace de tête ;
des moyens d'aspiration (18) pour aspirer l'égalisation de vapeur dans la pile à combustible ; et
le collecteur est configuré de sorte que l'air provenant de l'extérieur de l'appareil de détection est aspiré à travers au moins une ouverture (24) dans le collecteur à travers au moins une partie de l'insert dans l'espace de tête lorsque l'égalisation de vapeur est aspirée dans la pile à combustible.

2. Un appareil de détection selon la revendication 1, dans lequel la chambre comprend au moins une ouverture (20) à travers laquelle de l'air provenant de l'extérieur de la chambre est aspiré dans l'espace de tête en même temps que l'égalisation de vapeur est aspirée dans la pile à combustible.

3. Un appareil de détection selon la revendication 2, dans lequel l'air est aspiré depuis un corps (22) de l'appareil de détection.

4. Un appareil de détection selon l'une quelconque des revendications précédentes, dans lequel la chambre et le collecteur sont tous deux configurés pour permettre à l'air d'être aspiré dans l'espace de tête lorsque l'égalisation de vapeur est aspirée dans la pile à combustible, la chambre comprenant au moins une ouverture (20) à travers laquelle de l'air provenant de l'extérieur de la chambre est aspiré dans l'espace de tête en même temps que l'égalisation de vapeur est aspirée dans la pile à combustible, et le collecteur comprend au moins une ouverture à travers laquelle de l'air provenant de l'extérieur de l'appareil de détection est aspiré dans l'espace de tête en même temps que l'égalisation de vapeur est aspirée dans la pile à combustible.

5. Un appareil de détection selon l'une quelconque des revendications précédentes, dans lequel l'appareil de détection a une minuterie pour permettre à l'échantillon d'équilibrer son niveau d'alcool avec l'air dans l'espace libre selon les lois universelles des gaz.

6. Un appareil de détection selon l'une quelconque des revendications précédentes, dans lequel l'appareil de détection comprend des moyens de conditionnement pour conditionner en température l'échantillon dans une égalisation de vapeur avec de l'air dans l'espace de tête, les moyens de conditionnement en température comprenant des éléments chauffants.

7. Un appareil de détection selon l'une quelconque des revendications précédentes, dans lequel l'appareil de détection comprend un capteur électrochimique, le capteur électrochimique étant pour le glucose sanguin.

8. Un appareil de détection selon l'une quelconque des revendications précédentes, l'appareil de détection comprenant un contrôleur ayant une pluralité d'algorithmes et/ou de profils de réponse pour les différentes conditions de température mettant en équivalence la tension électrique à chaque condition de température à la teneur en alcool dans l'égalisation de vapeur de l'échantillon et l'air dans l'espace de tête.

9. Un appareil de détection selon l'une quelconque des revendications précédentes, dans lequel l'appareil de détection est configuré de sorte qu'il se purge lui-même afin d'être prêt pour l'opération d'analyse suivante.

10. Un appareil de détection selon l'une quelconque des revendications précédentes, dans lequel les moyens de tirage sont des soufflets ou une pompe volumétrique.

11. Un procédé de détection d'alcool dans un échantillon en utilisant un appareil de détection (10), le procédé comprenant :
le fait d'associer un collecteur (100) à une chambre (12), de sorte que la chambre reçoit au moins une partie du collecteur, le collecteur comprenant :
un échantillon ;
une structure (102) ayant un évidement (104) à une extrémité dans lequel un insert (106) comprenant un matériau absorbant pour recevoir l'échantillon peut être placé ; et
une gaine de protection (108) pour recouvrir sensiblement l'insert à l'exception d'une zone destinée à recevoir l'échantillon ;
le fait de contenir une égalisation de vapeur provoquée par la vapeur provenant de l'échantillon s'équilibrant avec l'air dans un espace de tête (14) dans la chambre lorsque ladite au moins une partie du collecteur est reçue dans la chambre ;
le fait d'aspirer l'égalisation de vapeur dans une pile à combustible (16) associée à l'espace de tête à l'aide de moyens d'aspiration (18) ; et
le fait d'aspirer de l'air par au moins une ouverture (24) du collecteur à travers au moins une partie de l'insert dans l'espace de tête au fur et à mesure que l'égalisation de vapeur est aspirée dans la pile à combustible.
